# EUROPÄISCHE PATENTSCHRIFT

(11) **EP 0 840 568 B1**
(45) Veröffentlichungstag und Bekanntmachung des Hinweises auf die Patenterteilung: **13.12.2000**
(21) Anmeldenummer: 96927049.5
(22) Anmeldetag: 24.07.1996
(51) Int. Cl.: A61B 5/12

(54) **ERMITTLUNG VON DATEN ÜBER DAS HÖRVERMÖGEN**
DETERMINATION OF DATA CONCERNING A PERSON'S AUDITORY CAPACITY
DETERMINATION DE DONNEES SUR LA CAPACITE AUDITIVE

(30) Priorität: 25.07.1995 DE 19527108
(43) Veröffentlichungstag der Anmeldung: 13.05.1998
(73) Patentinhaber: Zenner, Hans Peter, Prof. Dr. med., 72070 Tübingen (DE); Gummer, Anthony, W., 72070 Tübingen (DE)
(72) Erfinder: Zenner, Hans Peter, Prof. Dr. med., 72070 Tübingen (DE); Gummer, Anthony, W., 72070 Tübingen (DE)
(74) Vertreter: Patentanwälte Ruff, Beier und Partner
(86) Internationale Anmeldenummer: EP9603258
(87) Internationale Veröffentlichungsnummer: WO9704706

(56) Entgegenhaltungen:
- EP-A- 0 084 972
- WO-A-89/01315
- WO-A-93/21820
- APPLIED OPTICS, Bd. 33, Nr. 4, 1.Februar 1994, NEW YORK US, Seiten 702-708, XP000429132 C.J. KOESTER ET AL.: "Confocal slit divided-aperture microscope: applications in ear research"
- HNO, Bd. 41, Nr. 1/93, Januar 1993, DE, Seiten 1-6, XP000603037 N. STASCHE ET AL.: "Laser-Doppler-Vibrometrie (LDV) des Trommelfells" in der Anmeldung erwähnt
- JOURNAL OF BIOMECHANICAL ENGINEERING, Bd. 101, Nr. 4, November 1979, NEW YORK (US), Seiten 267-270, XP002022019 J.M. HAMELINK ET AL.: "Ocular Tonometry Through Sonic Excitation and Laser Doppler Velocimetry"
- American Journal of Ontology Band 14 Nummer 3 May 1993 Seite 247
- Hearing Research Band 51 1991 Elseviers Seite 203 - 214
- Fiber Optic Laser Vibrometer Broschüre der Firma Polytec Optronics , Costa Mesa , USA

## Beschreibung

Die Erfindung betrifft ein Verfahren zur Ermittlung von Daten über das Hörvermögen.

Es wurden bereits umfangreiche Untersuchungen vorgenommen, um qualitative und insbesondere auch quantitative Ergebnisse über den Zustand des normalen und des gestörten Gehörs zu erhalten. In diesem Zusammenhang ist insbesondere die Entdeckung von Kemp (s. EP-B1-15258) von Bedeutung, daß als Reaktion auf ein Schallereignis Schallemissionen im äußeren Gehörgang gemessen werden können, die mit dem Zustand im Ohr im Zusammenhang stehen. Diese sogenannten evozierten otoakustischen Emissionen wurden durch Kemp mit Hilfe einer akustischen Sonde, bestehend aus einem hochempfindlichen Miniaturmikrofon und einem Schallsender gemessen. Es ist auch bekannt, daß otoakustische Emissionen spontan, d.h. ohne Reizung des Ohres von außen, entstehen können.

Direkte Schwingungsmessungen am Trommelfell zur Untersuchung der Mittelohrmechanik werden bereits mit der Methode der Laser-Doppler-Vibrometrie durchgeführt. Diese Methode ist in der Veröffentlichung von N. Stasche, H.-J. Foth und K. Hörmann in HNO (1993), 41, Seiten 1 bis 6 beschrieben. Dabei wird der Strahl eines He-Ne-Lasers durch Strahlteiler aufgespalten und die beiden Teilstrahlen, von denen einer auf das zu untersuchende Objekt gelenkt wird, anschließend zur Interferenz gebracht. Aus der Frequenzverschiebung wird die Amplitude des gemessenen Trommelfellpunktes bestimmt. Zur Einkopplung des Laserlichts auf das zu untersuchende Objekt wird bei Stasche et al. eine flexible Lichtleitfaser benutzt, die in unmittelbare Nähe dieses Objekts gebracht werden muß.

Dies kann neben Auflösungsproblemen (kleine Apertur) auch zu Schwierigkeiten bei einer Messung am Patienten führen, da die Lichtleitfaser das Trommelfell nicht berühren darf.

Charles J. Koester et. al beschreiben in Applied Optics, Bd. 33 (1994), S. 702-708 eine invasive Methode, bei welcher das ganze Schläfenbein entfernt und entsprechend präpariert wird. Das verwendete Mikroskop zeigt insgesamt einen komplizierten Aufbau, da hauptsächlich Details einzelner Zellen sichtbar gemacht werden sollen. Obwohl grundsätzlich die Möglichkeit zur Vibrationsmessung besteht, ist die Apparatur, nicht zuletzt wegen ihres aufwendigen Aufbaus, nicht für einen routinemäßigen klinischen Einsatz für die Messung am Trommelfell geeignet.

In einer Veröffentlichung von Alfred L. Nuttall et al in Hearing Research Bd. 51 (1991), S. 203-214 werden Messungen direkt an der Basilarmembran beschrieben. Die Messungen erfolgen am zwar lebenden, jedoch invasiv präparierten Tier und sind dementsprechend nicht auf Messungen am Trommelfell lebender Patienten übertragbar. Weiter ist zur Durchführung der Messung ein Aufbringen von Mikroglasperlen auf das Meßobjekt erforderlich, was die Apparatur für einen routinemäßigen klinischen Einsatz weitgehend unbrauchbar macht.

Richard L. Goode et al in The American Journal of Otology, Bd. 14, (1993), S. 247-251 zeigen einen weiteren Ansatz, um Messungen am Trommelfell für einen möglichen klinischen Einsatz anwendbar zu machen. Hierfür wird ein Gelenkarm zur Herstellung einer bewegbaren Apparatur eingesetzt. Dies führt jedoch auch hier zu Intensitätsverlusten und dementsprechend unbefriedigenden Signal-Rausch-Verhältnissen. Deshalb muß vor Durchführung der Messung mit Hilfe von einer Art Vaseline ein Stück eines Reflexionsbandes auf das Trommelfell aufgebracht werden, um eine ausreichende Reflexion des eingestrahlten Lichts zu erreichen. All dies zusammen erschwert den routinemäßigen klinischen Einsatz.

Die Erfindung stellt sich die Aufgabe, die Bestimmung des Hörvermögens, insbesondere durch Ermittlung quantifizierbarer Daten, weiter zu verbessern. Dabei sollen die beschriebenen Nachteile des Standes der Technik vermieden werden. Insbesondere soll ein Verfahren zur Verfügung gestellt werden, durch das eine einfache, für den Patienten weitgehend belastungsfreie Bestimmung der Meßdaten möglich wird. Weiterhin soll das Verfahren geeignet sein, ggf. nach einem Auswerteschritt, Informationen des Innenohres zu erhalten, die einem Arzt als Grundlage für eine Diagnose und/oder Therapie dienen können.

Diese Aufgaben werden gelöst durch ein Verfahren mit den Merkmalen von Anspruch 1. Eine bevorzugte Ausführungsform des Verfahrens ist in dem abhängigen Anspruch 2 beschrieben. Der Wortlaut sämtlicher Ansprüche wird durch Bezugnahme zum Inhalt der Beschreibung gemacht.

Das erfindungsgemäße Verfahren zur Ermittlung von Daten über das Hörvermögen verwendet Mittel zur, insbesondere berührungsfreien Messung von Vibrationen des Mittelohres und/oder des Trommelfells mit Hilfe von elektromagnetischen Wellen auf. Dabei ist zur Einkopplung der zur Messung verwendeten elektromagnetischen Wellen auf das Meßobjekt ein Mikroskop, insbesondere ein optisches Mikroskop, vorgesehen.

Die Einkopplung erfolgt in der Weise, daß die elektromagnetischen Strahlen durch das optische System des Mikroskops hindurch auf das Meßobjekt geführt werden. Die vom Meßobjekt reflektierten Strahlen werden vorzugsweise ebenfalls durch das optische System des Mikroskops zurückgeführt und anschließend analysiert. Durch die Meßanordnung können im Vergleich zu bekannten Vorrichtungen höhere Genauigkeiten erzielt werden. Dadurch werden Ereignisse im Ohr detektierbar und quantifizierbar, die einer Messung bisher nicht zugänglich waren. Dies ist unter anderem darauf zurückzuführen, daß ein großer Teil der reflektierten elektromagnetischen Wellen in den Analysator geführt werden kann (hohe Apertur) und dementsprechend das Signal/Rausch-Verhältnis verbessert ist.

Wie beschrieben ist das erfindungsgemäße Verfahren zur Messung von Vibrationen des Mittelohres und/oder des Trommelfells geeignet. Damit soll in erster Linie zum Ausdruck gebracht werden, daß die Vibrationsmessung am Trommelfell und grundsätzlich an allen Ossikeln möglich ist. Dabei wird die (ambulante) Ermittlung von Meßdaten üblicherweise am Trommelfell erfolgen, da in diesem Fall die Bestimmung des Hörvermögens keinen (chirurgischen) Eingriff erfordert.

Bei dem erwähnten Mikroskop handelt es sich zweckmäßig um ein sogenanntes Ohrmikroskop, d.h. um ein in diesem Bereich standardmäßig verwendetes Gerät. Sowohl in diesen Fällen als auch allgemein kann ein modifiziertes Operationsmikroskop in dem erfindungsgemäßen Verfahren eingesetzt werden.

Das Mikroskop ist vorzugsweise modular aufgebaut, d.h. es besteht aus mehreren Bauteilen, die auf den jeweiligen Anwendungsfall abgestimmt zusammengesetzt werden können. Ein solches modular aufgebautes Mikroskop weist vorzugsweise ein austauschbares Modul für die Einkopplung der elektromagnetischen Wellen auf. Dadurch kann das Mikroskop sowohl für die Erfindung als auch für andere Einsatzmöglichkeiten verwendet werden.

In Weiterbildung besitzt das Mikroskop zur Einkopplung der elektromagnetischen Wellen mindestens einen Spiegel, insbesondere einen Umlenkspiegel. Bei den beschriebenen bevorzugten Ausführungsformen ist dieser Spiegel in dem austauschbaren Modul vorgesehen. Der Spiegel dient dazu, die in das Mikroskop eingestrahlten elektromagnetischen Wellen, ggf. unter Umlenkung, durch das (optische) System auf das Meßobjekt zu lenken. In entsprechender Weise kann der Spiegel dazu dienen, die reflektierten Strahlen wieder aus dem Mikroskop herauszuführen.

Bei dem erfindungsgemäß Verfahren erfolgt die Messung der Vibrationen mit Hilfe kohärenter elektromagnetischer Strahlung. Dadurch wird die Messung und Auswertung vereinfacht. Insbesondere ist zur Messung Laserstrahlung vorgesehen, beispielsweise aus einem Helium-Neon-Laser. Ein derartiger Laser arbeitet beispielsweise bei einer Wellenlänge von 633 nm.

Die Mittel zur Messung der Vibrationen umfassen insbesondere ein Interferometer, d.h. ein Gerät, mit dessen Hilfe Interferenzerscheinungen zwischen den eingestrahlten elektromagnetischen Wellen und den reflektierten elektromagnetischen Wellen bestimmt werden. Bei einem derartigen Interferometer handelt es sich vorzugsweise um ein übliches sog. Vibrometer, dessen Aufbau und Funktion dem Fachmann bekannt ist. Bei der Messung mit Lasern sind die bekannten Laser-Vibrometer, insbesondere die sog. Laser-Doppler-Vibrometer einsetzbar, wie sie beispielsweise in der oben erwähnten Veröffentlichung von Stasche et al. beschrieben sind. Insoweit wird der Inhalt dieser Veröffentlichung zum Bestandteil dieser Beschreibung gemacht.

Wie bereits angedeutet, kann das erfindungsgemäße Verfahren dazu vorgesehen sein, spontan auftretende Ereignisse im Ohr, insbesondere im Innenohr, mit Hilfe der Vibrationsmessung zu detektieren. Um reproduzierbare und vergleichbare Ergebnisse zu erhalten, können die Ereignisse auch durch einen (äußeren) Einfluß hervorgerufen, d.h. evoziert sein. In diesen Fällen weist eine entsprechende Vorrichtung vorzugsweise Stimulationsmittel auf, mit deren Hilfe die Ereignisse im Ohr, insbesondere im Innenohr, beispielsweise durch Übertragung von Reizen von außen hervorrufbar sind.

Die genannten Stimulationsmittel können möglichst nahe an das Trommelfell und/oder die Ossikel des Mittelohres herangeführt sein, um eine möglichst effiziente Reizübertragung zu bewirken. Dabei können geeignete Übertragungseinrichtungen vorgesehen sein, die den "Reiz" an den entsprechenden Teil des Mittelohres oder des Trommelfells heranführen. Bei derartigen Übertragungseinrichtungen kann es sich beispielsweise um Kanülen, Drähte oder gleichwirkende Einrichtungen handeln. Um Schädigungen des jeweiligen Meßobjektes zu vermeiden, sollten die Stimulationsmittel bzw. die Übertragungseinrichtungen das Meßobjekt möglichst nicht berühren. Dazu können geeignete Sicherungsmittel vorgesehen sein, die die Übertragungseinrichtung in einem Abstand zum Meßobjekt festlegen. Dies gilt zumindest dann, wenn die Messung am lebenden Objekt erfolgt. Sofern dies nicht der Fall ist, kann es bevorzugt sein, wenn die Stimulationsmittel bzw. die Übertragungseinrichtungen mit dem Trommelfell oder den Ossikeln direkt in Kontakt stehen. Dadurch wird eine verbesserte Reizübertragung erreicht.

Bei bevorzugten Ausführungsformen sind die Stimulationsmittel zur akustischen Reizung ausgebildet. Dabei weist das Stimulationsmittel insbesondere eine Schallquelle auf, die vorzugsweise im äußeren Gehörgang anordenbar ist oder mit dem äußeren Gehörgang in Verbindung steht. Bei dem akustischen Reiz kann es sich beispielsweise um ein "Geräusch", d.h. um eine Superposition "unendlich" vieler Töne handeln. Um definierte Schallstimuli vorzusehen, können auch insbesondere zwei gleichzeitig präsentierte Sinustöne benutzt werden, die die Frequenzen f₁ und f₂ aufweisen. Dadurch werden im Innenohr Emissionen evoziert, insbesondere die sogenannten 2f₁-f₂- und höhere Distorsionsprodukte, die dann durch Vibrationsmessung am Trommelfell oder den Ossikeln detektiert werden. In Abwandlung können auch mehr als zwei Sinustöne bzw. Sinussignale, d.h. ein sog. Multisinussignal zur Stimulierung verwendet werden.

Bei anderen bevorzugten Ausführungsformen ist das Stimulationsmittel zur mechanischen Stimulation des Mittelohres ausgebildet. Es kann insbesondere ein sogenannter piezoelektrischer Wandler vorgesehen sein. Dabei kann der Zuleitungsdraht dieses Wandlers ggf. zusätzlich in einer Kanüle auf das Piezoelement geführt sein. Gegebenenfalls ist ein solches Stimulationsmittel direkt an einem Ossikel, insbesondere am sogenannten Steigbügel, montiert.

Bei anderen bevorzugten Ausführungsformen ist das Stimulationsmittel zur (direkten) elektrischen Reizung ausgebildet, wobei insbesondere eine Elektrode vorgesehen ist. Diese Elektrode kann beispielsweise direkt auf die Cochlea im Innenohr geführt sein.

In Weiterbildung weist eine entsprechende Vorrichtung Mittel zum Auswerten der Meßsignale auf, die bei der Messung der Vibrationen des Mittelohres und/oder des Trommelfells erhalten werden. Neben solchen Auswertemitteln sind selbstverständlich entsprechende Detektoren für die elektromagnetischen Strahlen, insbesondere Laserdetektoren und Einrichtungen zur Aufbereitung der spektralen Daten, insbesondere sogenannte Laser-Dekoder zur Aufbereitung der Meßsignale vorhanden.

Die genannten Auswertemittel umfassen vorzugsweise einen sogenannten Analysator, insbesondere einen Software-Analysator, mit dessen Hilfe aus dem erhaltenen und ggf. aufbereiteten Meßsignal die Anteile des Innenohres ableitbar sind. Das erhaltene Meßsignal besteht nämlich im wesentlichen aus drei Anteilen, die das auf das Meßobjekt eingestrahlte Eingangssignal, die "Mittelohr-Komponente" und die "Innenohr-Komponente" repräsentieren. Deshalb soll der Analysator, insbesondere der Software-Analysator, in der Lage sein, den auf das Eingangssignal zurückzuführenden Anteil zu berücksichtigen und ggf. aus dem verbleibenden "Restsignal" die auf das Mittelohr oder das Innenohr zurückzuführenden Anteile abzuleiten. Dies kann insbesondere durch eine geeignete Software erreicht werden, in die ggf. für die einzelnen zu eliminierenden Anteile Modellvorstellungen oder experimentelle Erfahrungswerte eingehen.

Weiterhin ist Teil der Erfindung die Verwendung der beschriebenen Vorrichtung oder des Mikroskops zur Detektion von im Innenohr stattfindenden Ereignissen. Durch diese Verwendung wird erstmals eine Messung ermöglicht, die derartige Ereignisse detektieren kann bzw. aus den erhaltenen Daten ableitbar macht. Vorzugsweise handelt es sich bei den im Innenohr stattfindenden Ereignissen um sogenannte mikromechanische Ereignisse. Dieser Begriff umfaßt alle Ereignisse, die im weitesten Sinne auf die Erzeugung mechanischer Energie im Innenohr zurückführbar sind. In diesem Zusammenhang wird auch von den sogenannten otoakustischen Emissionen (OAE) gesprochen. Wie bereits beschrieben, können diese spontan auftreten oder evoziert werden. Da sie auch durch Pharmaka gehemmt oder unterdrückt werden können, ist eine genaue Untersuchung dieser Effekte von besonderer Bedeutung.

Die Erfindung betrifft ein Verfahren zur Ermittlung von Daten über das Hörvermögen, das sich insbesondere einer beschriebenen Vorrichtung bedient. Bei einem solchen Verfahren werden Vibrationen des Mittelohres (seiner Ossikel) und/oder des Trommelsfells mit Hilfe elektromagnetischer Wellen vorzugsweise berührungsfrei gemessen. Aus den erhaltenen Meßsignalen, die wie erwähnt insbesondere im wesentlichen aus einem Anteil des Eingangssignals, einem Anteil des Mittelohres und einem Anteil des Innenohres zusammengesetzt sind, werden in mindestens einem Auswerteschritt die Anteile des Innenohres an diesem Gesamtsignal ermittelt. Bei diesem Verfahren wird der Anteil des Innenohres ermittelt, d.h. es wird erstmals der auf Ereignisse im Innenohr zurückzuführende Anteil des gemessenen Gesamtsignals bestimmt.

Die Ermittlung einzelner Anteile kann auf verschiedene Weise erfolgen. Für bestimmte Anwendungsfälle kann es ausreichen, wenn vom Gesamtsignal lediglich der Anteil des Eingangssignals abgezogen wird. In diesen Fällen können bereits aus dem verbleibenden Anteil (Mittelohr und Innenohr) nützliche Daten erhalten werden, unter Umständen auch ein Anteil des Innenohres abgeleitet werden. Erfindungsgemäß wird vom Gesamtsignal sowohl der Anteil des Eingangssignals als auch ein auf das Mittelohr zurückzuführender Anteil abgezogen. Dadurch wird der auf das Innenohr zurückgehende Anteil erhalten. In diesem Zusammenhang ist der Ausdruck "Abziehen" im Zusammenhang mit der üblichen Fourier-Transformation der Meßdaten zu verstehen, wobei die spektralen Daten in einer dem Fachmann bekannten Weise aufgearbeitet werden. Selbstverständlich können die einzelnen Anteile durch bekannte gleichwertige Verfahren voneinander separiert werden.

Das erfindungsgemäße Verfahren kann wie erwähnt unter Verwendung der beschriebenen Vorrichtung durchgeführt werden, wobei insbesondere zur Ankopplung der elektromagnetischen Wellen ein optisches Mikroskop eingesetzt wird.

Vorzugsweise wird bei dem erfindungsgemäßen Verfahren zur Messung der Vibration ein einzelner Strahl der elektromagnetischen Wellen, insbesondere ein Laserstrahl auf das Trommelfell und/oder mindestens ein Ossikel des Mittelohres eingestrahlt.

In Weiterbildung ist das Verfahren dadurch gekennzeichnet, daß Laserlicht durch ein optisches Mikroskop auf das Trommelfell aufgestrahlt wird, der reflektierte Laserstrahl mit dem eingestrahlten Laserstrahl zur Interferenz gebracht wird, die erhaltenen Meßdaten in üblicher Weise dekodiert (aufgearbeitet) und anschließend die Anteile des Innenohres am erhaltenen Gesamtsignal ermittelt werden. Bei dem verwendeten Laser handelt es sich insbesondere um einen Helium-Neon-Laser. Der reflektierte Laserstrahl wird vorzugsweise ebenfalls durch das optische Mikroskop hindurchgeführt und anschließend mit dem eingestrahlten Laserstrahl zur Interferenz gebracht. Bei Verwendung eines Laser-Vibrometers erfolgt die Strahlaufteilung im Vibrometer und die Interferenz der einzelnen Strahlen wird im Detektorteil dieses Vibrometers vorgenommen.

Auch hier ist das Verfahren vorzugsweise so ausgestaltet, daß vom erhaltenen Gesamtsignal der Anteil des Eingangssignals abgezogen und aus dem so erhaltenen Endsignal Charakteristika des Innenohres, ggf. durch Abzug der auf das Mittelohr zurückzuführenden Signalkomponente, abgeleitet werden. Für das Abziehen des Mittelohranteils können Daten aus mindestens einem (mathematischen) Modell und/oder Erfahrungsdaten, beispielsweise aus klinischen Versuchen, verwendet werden, die insbesondere die Bewegung der Ossikel im hörbaren Bereich berücksichtigen bzw. repräsentieren.

Wie bereits erwähnt, wird bei den beschriebenen Verfahren zumindest der auf das Innenohr zurückgehende (Gesamt)Anteil des Meßsignals ermittelt. Dieser wird durch Abzug des Mittelohranteils erhalten. Dies ist einerseits wichtig für grundsätzliche Funktionsuntersuchungen des Ohres und andererseits auch Voraussetzung für eine effektive Behandlung von Krankheiten, die mit einer Funktionsstörung des Innenohres in Zusammenhang gebracht werden, wie beispielsweise Morbus Meniere.

Hier ist von besonderer Bedeutung, daß es die Erfindung u.a. ermöglicht, über eine Messung am Trommelfell Informationen über das Innenohr zu erhalten. Eine Messung am Trommelfell ist nämlich beispielsweise in der Poliklinik mit vergleichsweise geringem Aufwand möglich.

Die beschriebenen Merkmale und weitere Merkmale der Erfindung ergeben sich aus der nachfolgenden Beschreibung von bevorzugten Ausführungsformen in Verbindung mit dem Unteranspruch und der Zeichnung. Hierbei können die einzelnen Merkmale jeweils für sich oder zu mehreren in Kombination miteinander verwirklicht sein.

In den Zeichnungen zeigen:

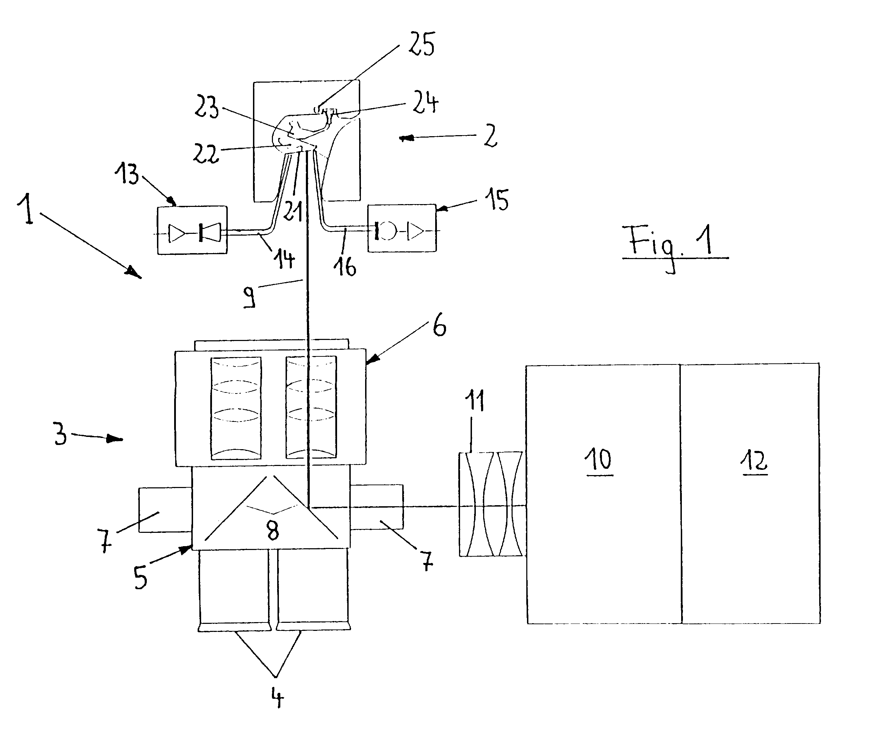

- Fig. 1: eine schematische Darstellung einer Vorrichtung die zur Durchführung des erfindungsgemäßen Verfahrens zur Messung des Hörvermögens an einem Meßobjekt geeignet ist.

In Fig. 1 ist eine Vorrichtung 1 dargestellt, die zur Durchführung des erfindungsgemäßen Verfahrens zur Ermittlung von Meßdaten an einem Meßobjekt 2 vorgesehen ist.

Die Vorrichtung 1 weist ein optisches Mikroskop 3 auf, das im wesentlichen aus einem Binokular 4, einem Modul 5 zur Einkopplung von Laserstrahlen und einem Operationsmikroskop-Kopf 6 aufgebaut ist. Das Modul 5 besitzt zwei Einlaßeinrichtungen 7 mit jeweils zugeordneten Umlenkspiegeln 8, mit deren Hilfe jeweils ein Laserstrahl in das Mikroskop 3 einkoppelbar ist. Im dargestellten Fall ist über die rechte Einlaßeinrichtung 7 und den rechten Umlenkspiegel 8 ein Laserstrahl 9 durch die nicht näher bezeichnete rechte Optik des Kopfes 6 eingekoppelt und auf das Meßobjekt 2 geführt. Durch das Binokular 4 kann der Laserstrahl auf die gewünschte Stelle am Meßobjekt 2 ausgerichtet werden.

Weiter ist in Fig. 1 ein Laser-Doppler-Vibrometer 10 schematisch dargestellt, das über ein Fokussierobjektiv 11 der rechten Einlaßeinrichtung 7 am Modul 5 zugeordnet ist. Das Laser-Doppler-Vibrometer (LDV) 10 ist aus dem eigentlichen Vibrometer und einem Laser-Detektor, die beide nicht näher dargestellt sind, aufgebaut. Bei dem LDV 10 handelt es sich um ein übliches Gerät. An das LDV 10 schließt sich eine Auswerteeinheit 12 an, die in Fig. 1 ebenfalls nur schematisch dargestellt ist. Diese Auswerteeinheit 12 ist im wesentlichen aus einem Analysator für das aus dem LDV 10 erhaltene Spektrum, der die Meßdaten dekodiert, und einem Software-Analysator aufgebaut.

Das in Fig. 1 dargestellte Laser-Doppler-Vibrometer 10 soll unter Verwendung eines He-Ne-Lasers mit einer Wellenlänge von 633 nm arbeiten. An die Auswerteeinheit 12 kann sich beispielsweise ein Computer oder eine Datenverarbeitung zur Steuerung der Vorrichtung oder zur weiteren Bearbeitung oder Darstellung der erhaltenen Meßdaten anschließen.

Weiter zeigt Fig. 1 unterhalb des Meßobjektes 2 eine Schallquelle 13 als Stimulationsmittel zur akustischen Evokation. Die Schallquelle 13 steht mit dem Meßobjekt 2 über eine Übertragungseinrichtung 14, beispielsweise in Form einer Kanüle in Verbindung. Außerdem ist dem Meßobjekt 2 über eine entsprechende Übertragungseinrichtung 16 ein (Sonden)Mikrofon 15 zugeordnet, mit dem der durch die Schallquelle 13 erzeugte Schalldruck gemessen wird. Auf diesen Wert wird dann der erhaltene Meßwert für die Vibration bezogen, beispielsweise Um/s, bezogen auf Pascal. Bei einer Evokation mit Hilfe einer Elektrode würde der erhaltene Vibrationswert in entsprechender Weise auf Ampere bezogen. Sowohl in der Darstellung der Schallquelle 13 als auch in der des Sondenmikrofons 15 sind nicht näher bezeichnete Verstärker für die entsprechenden Signale zeichnerisch angedeutet. Zur Festlegung der Übertragungseinrichtungen 14, 16, z.B. der Kanülen können Sicherungsmittel beispielsweise in Form von konusartigen Halteelementen vorgesehen sein, die verhindern, daß die Übertragungseinrichtungen 14, 16 das Meßobjekt 2 berühren, und diese ggf. in definiertem Abstand zum Meßobjekt 2 fixieren.

Das Meßobjekt 2 ist in Fig. 1 so dargestellt, daß es schematisch das menschliche Ohr wiedergibt. Der Laserstrahl 9 ist auf das Trommelfell 21 gerichtet, an das sich Hammer 22, Amboß 23 und Steigbügel 24 anschließen. Schließlich ist noch das runde Fenster 25 angedeutet.

Das bereits beschriebene erfindungsgemäße Verfahren wird durch die Fig. 1 verdeutlicht. Der aus dem Laser-Doppler-Vibrometer 10 austretende Laserstrahl 9 wird nach Fokussierung über das Mikroskop 3 auf das Meßobjekt 2 eingekoppelt. Der reflektierte Laserstrahl 9 wird durch das Mikroskop 3 in gleicher Weise zurückgeführt und im LDV 10 (im Detektorteil) mit einem entsprechenden Teilstrahl zur Interferenz gebracht.

Die erhaltenen spektralen Meßdaten werden dekodiert und in der beschriebenen Weise durch den Software-Analysator aufgearbeitet und ggf. durch weitere Geräte dargestellt. Die Steuerung der Anlage kann durch einen Computer erfolgen, wobei insbesondere bei Evokation durch die Schallquelle 13 ein Triggersignal übertragen wird.

## Patentansprüche

1. Verfahren zur Ermittlung von Daten über im Innenohr stattfindende Ereignisse, bei dem kohärente elektromagnetische Wellen aus einem Interferometer über ein diesem zugeordnetes Fokussierobjektiv in ein optisches Mikroskop eingekoppelt werden und durch das optische Mikroskop auf ein Ossikel des Mittelohrs und/oder auf das Trommelfell aufgestrahlt werden, reflektierte Wellen mit den eingestrahlten Wellen zur Interferenz gebracht werden und aus dem so erhaltenen Meßsignal, gegebenenfalls nach Abziehen des Eingangssignals, durch Abziehen des Anteils des Mittelohrs der Anteil des Innenohrs am Meßsignal bestimmt wird, wobei der Anteil des Mittelohrs aus Modelldaten und/oder Erfahrungsdaten, die insbesondere die Bewegung der Ossikel im hörbaren Bereich repräsentieren, gebildet ist.

2. Verfahren nach Anspruch 1, dadurch gekennzeichnet, daß es sich bei den kohärenten elektromagnetischen Wellen um Laserstrahlung, vorzugsweise Strahlung eines Helium-Neon-Lasers, handelt.

## Claims

1. A method for determination of data concerning events taking place in the inner ear in which coherent electromagnetic waves from an interferometer are coupled via a focussing objective lens assigned thereto into an optical microscope and irradiated by the optical microscope onto an ossicle of the middle ear and/or to the ear drum, reflected waves being brought to interference with the irradiated waves and determining from the resulting measurement signal, where necessary following deduction of the input signal, the contribution made by the inner car in the measurement signal by deducting the contribution made by the middle ear, the contribution made by the middle ear being formed from model data and/or data gained from experience, representing more particularly the movement of the ossicles in the audible range.

2. The method as set forth in claim 1, characterized in that said coherent electromagnetic waves are laser radiation, preferably radiation of a helium-neon laser.

## Revendications

1. Procédé pour la détermination de données concernant des événements se produisant dans le conduit auditif, où des ondes cohérentes électromagnétiques émises par un interféromètre sont couplées par l'intermédiaire d'un objectif de focalisation associé à l'interféromètre dans un microscope optique et sont envoyées par le microscope optique sur un ossicule de l'oreille moyenne et/ou sur le tympan, les ondes réfléchies étant amenées en interférence avec les ondes émises et à partir du signal de mesure en résultant, le cas échéant après la soustraction du signal d'entrée, la part de l'oreille interne étant déterminée par soustraction de la part de l'oreille moyennej, la part de l'oreille moyenne étant constituée par des données modèles et/ou des données expérimentales, qui représentent en particulier le mouvement des ossicules dans la zone audible.

2. Procédé selon la revendication 1, caractérisé en ce que pour les ondes cohérentes électromagnétiques, il s'agit d'un rayonnement laser, de préférence d'un rayonnement d'un laser à hélium et néon.
